# EUROPÄISCHE PATENTSCHRIFT

(11) **EP 3 560 410 B1**
(45) Veröffentlichungstag und Bekanntmachung des Hinweises auf die Patenterteilung: **19.10.2022**
(21) Anmeldenummer: 19169707.7
(22) Anmeldetag: 17.04.2019
(51) Int. Cl.: A61B 1/00

(54) **ANORDNUNG ZUR STERILEN HANDHABUNG EINES NICHT STERILEN ENDOSKOPS IN EINER STERILEN UMGEBUNG**
ARRANGEMENT FOR STERILE HANDLING OF A NON-STERILE ENDOSCOPE IN A STERILE ENVIRONMENT
DISPOSITIF POUR LA MANIPULATION STÉRILE D'UN ENDOSCOPE NON STÉRILE DANS UN ENVIRONNEMENT STÉRILE

(30) Priorität: 26.04.2018 DE 102018110095
(43) Veröffentlichungstag der Anmeldung: 30.10.2019
(73) Patentinhaber: avateramedical GmbH, 07745 Jena (DE)
(72) Erfinder: Preuß, Peter, 15537 Gosen-Neu Zittau (DE)
(74) Vertreter: Schaumburg und Partner Patentanwälte mbB

(56) Entgegenhaltungen:
- WO-A1-2015/187626
- DE-A1- 2 932 116
- DE-A1-102016 007 669
- US-A1- 2005 283 048
- US-A1- 2013 218 147
- US-A1- 2015 018 613

## Beschreibung

Die Erfindung betrifft eine Anordnung zur sterilen Handhabung eines nicht sterilen Endoskops in einer sterilen Umgebung umfassend eine sterile Endoskophülle.

Bekannte Anordnungen zur sterilen Handhabung eines nicht sterilen Endoskops in einer sterilen Umgebung, wie einem Operationssaal, umfassen ein nicht steriles Endoskop und eine sterile Endoskophülle, die ein an einem distalen Ende angeordnetes optisches Element hat. Bei der Endoskophülle handelt es sich typischerweise um einen sterilen Einmalartikel oder um eine Endoskophülle, die wieder sterilisiert, d.h. wiederaufbereitet, werden kann. Eine solche Endoskophülle ist beispielsweise aus dem Dokument DE 10 2010 022 429 A1 bekannt. Die Endoskophülle hat zwei Hüllenteile, die lösbar und fluiddicht mechanisch miteinander verbindbar sind. Dokument DE 10 2010 053 814 A1 offenbart ferner ein Endoskop für medizinische Zwecke, das in ein sterilisiertes Gehäuse eingesetzt werden kann.

Aus dem Dokument EP 0 904 725 A1 ist ein Endoskop mit einem auswechselbaren Schaft bekannt, der als steriler Einmalartikel ausgebildet ist. Nachteilig bei diesem Endoskop sind die vergleichsweise hohen Kosten, die durch das Auswechseln des Schafts bei jeder Anwendung entstehen.

Das optische Element bekannter Endoskophüllen ist für sichtbares Licht transparent. Daher erfolgt eine Beleuchtung des Situs, d.h. des eröffneten Operationsgebiets, durch Beleuchtungslicht, das aus einem distalen Ende eines in der Endoskophülle angeordneten Endoskops abgestrahlt wird. Eine Verschmutzung des optischen Elements, beispielsweise durch Residuen, führt allerdings dazu, dass das optische Element zumindest in Teilbereichen für das Beleuchtungslicht undurchlässig wird und einen Teil des Beleuchtungslichts absorbiert. Das absorbierte Beleuchtungslicht wird in Wärme umgewandelt und bewirkt eine starke Erwärmung des optischen Elements, durch die es zu erheblichen Schädigungen an Organen kommen kann. Nachteilig bei bisher bekannten Endoskophüllen ist, dass diese Erwärmung nicht festgestellt werden kann.

Aus dem Dokument US 2014/0200406 A1 ist ein Endoskop bekannt, bei dem das Beschlagen eines distal angeordneten optischen Elements mithilfe von Infrarotlicht verhindert wird. Das optische Element ist derart ausgebildet, dass es das Infrarotlicht absorbiert. Durch die Absorption erwärmt sich das optisch Element, wodurch ein Beschlagen verhindert und/oder bereits vorhandenes Kondenswasser verdampft wird.

Aus dem Dokument DE 21 29 094 A1 ist eine Alarmeinrichtung zur Temperaturüberwachung von Uranbrennstäben in einem Speicherraum bekannt. Die Alarmeinrichtung hat eine Optik zum Auffangen von aus dem Speicherraum ausgehender elektromagnetischer Strahlung in Infrarotbereich und einen Infrarotdetektor mit einem vorgeschalteten Filter, der für einen infraroten Wellenlängenbereich durchlässig ist.

Ferner ist aus Dokument EP 0 820 250 B1 ein System zur endoskopischen Diagnose bekannt, das sowohl sichtbares als auch infrarotes Licht zur Bildgebung verwendet.

Ausgehend von dem bekannten Stand der Technik ist es Aufgabe der Erfindung, eine Endoskophülle anzugeben, bei der die Temperatur eines an einem distalen Ende der Endoskophülle angeordneten optischen Elements zuverlässig ermittelt werden kann. Darüber hinaus ist eine Anordnung zur sterilen Handhabung eines nicht sterilen Endoskops in einer sterilen Umgebung anzugeben.

Das Dokument WO 2015/187626 A1 offenbart eine Vorrichtung zur endoskopischen Temperaturmessung gemäß des Oberbegriffs des Anspruchs 1. Die Vorrichtung umfasst eine endoskopische Temperatursonde und eine sterile Hülle, in welche die Temperatursonde aufnehmbar ist. Das Dokument US 2013/028147 A1 offenbart eine sterile Schutzhülle für ein Endoskop. Das Dokument DE 2016 007 offenbart eine sterile Hülle für ein Endoskop. Die Hülle weist ein Heizelement auf, das ausgebildet ist, ein Beschlagen der Hülle zu verhindern. Ferner wird zum Stand der Technik auf das Dokument US 2015/283048 A1 verwiesen, das ein Endoskopsystem offenbart.

Diese Aufgabe wird durch eine Anordnung mit den Merkmalen des Anspruchs 1 gelöst. Vorteilhafte Weiterbildungen sind in den abhängigen Ansprüchen angegeben.

Die sterile Endoskophülle der erfindungsgemäßen Anordnung für ein nichtsteriles Endoskop hat ein an einem distalen Ende der Endoskophülle angeordnetes optisches Element. Das optische Element absorbiert elektromagnetische Strahlung in einem Absorptionswellenlängenbereich, der im mittleren infraroten Wellenlängenbereich liegt. Unter dem mittleren infraroten Wellenlängenbereich wird in diesem Dokument der Wellenlängenbereich zwischen 3 µm und 50 µm verstanden. Dieser Wellenlängenbereich entspricht dem Wellenlängenbereich von Wärmestrahlung bei auf der Erde vorhandenen Temperaturen. In diesem Dokument wird als distal eine dem Patienten zugewandte Richtung und als proximal eine dem Patienten abgewandte Richtung bezeichnet. Bei einem Bezug auf ein Element, einen Gegenstand oder eine Anordnung wird distal und proximal in Bezug auf die bestimmungsgemäße Lage des Elements, des Gegenstandes bzw. der Anordnung verwendet.

Da das optische Element die elektromagnetische Strahlung in dem Absorptionswellenlängenbereich absorbiert, hat das optische Element einen sehr geringen Reflexionsgrad und einen sehr hohen Emissionsgrad in dem Absorptionswellenlängenbereich. Das optische Element ist gleichsam ein geschlossener Hohlraum bzw. ein schwarzer Strahler für elektromagnetische Strahlung in dem Absorptionswellenlängenbereich. Damit entspricht die von dem optischen Element ausgehende elektromagnetische Strahlung in dem Absorptionswellenlängenbereich im Wesentlichen der von dem optischen Element ausgehenden Wärmestrahlung, die lediglich von der Temperatur des optischen Elements abhängig ist. Das optische Element ist ferner für die elektromagnetische Strahlung in dem Absorptionswellenlängenbereich undurchlässig. Quellen elektromagnetischer Strahlung in dem Absorptionswellenlängenbereich, wie beispielsweise Organe oder andere chirurgische Instrumente im Situs eines Patienten, werden durch das optische Element verdeckt. Somit ist es möglich, ausschließlich die durch das optische Element selbst abgestrahlte Wärmestrahlung mithilfe eines Sensors zu detektieren. Da aus dem Spektrum dieser Strahlung die Temperatur des optischen Elements ermittelt werden kann, ist es bei der erfindungsgemäßen Endoskophülle möglich, die Temperatur des optischen Elements zuverlässig zu ermitteln.

Es ist vorteilhaft, wenn das optische Element in wenigstens einem optischen Wellenlängenbereich außerhalb des Absorptionswellenlängenbereichs transparent ist. Vorzugsweise liegt der wenigstens eine optische Wellenlängenbereich im Bereich des sichtbaren Lichts, d.h. in einem Bereich von 380 nm bis 780 nm. Dies erlaubt es, das optische Element, beispielsweise in Verbindung mit einem Endoskop, für die Bildgebung im optischen Bereich zu verwenden, ohne dass es dabei zu Beeinträchtigungen bei der Bildgebung kommt.

Es ist ferner vorteilhaft, wenn der Absorptionswellenlängenbereich ein Wellenlängenbereich von 9 µm bis 10 µm, bevorzugt von 8 µm bis 12 µm, besonders bevorzugt von 8 µm bis 14 µm, ist. In dem Wellenlängenbereich von 9 µm bis 10µm liegen die Maxima von Wärmestrahlungsspektren schwarzer Körper mit den Temperaturen von 17°C bis 48°C. Durch eine Messung der von dem optischen Element ausgehenden elektromagnetischen Strahlung in diesem Wellenlängenbereich ist es möglich festzustellen, wenn sich das optische Element auf eine Temperatur oberhalb der Körpertemperatur von ca. 37°C erwärmt. In dem bevorzugten Wellenlängenbereich von 8 µm bis 12µm liegen die Maxima von Wärmestrahlungsspektren schwarzer Körper mit den Temperaturen von -31°C bis 89°C. Damit ist es möglich festzustellen, wenn sich das optische Element auf eine Temperatur oberhalb der Koagulationstemperatur von Gewebe, d.h. der Temperatur, ab der Proteine gerinnen, von ca. 60°C erwärmt. Erwärmt sich das Endoskop auf eine Temperatur oberhalb der Koagulationstemperatur, kann es zu Nekrose an Gewebe insbesondere von Organen kommen, mit dem bzw. denen das optische Element in Kontakt kommt. Der besonders bevorzugte Wellenlängenbereich von 8 µm bis 14µm umfasst die Maxima von Wärmestrahlungsspektren schwarzer Körper mit den Temperaturen von -60°C bis 89°C. Die Messung eines breiten Spektrums erlaubt eine noch zuverlässigere Bestimmung der Temperatur des optischen Elementes.

Die erfindungsgemäße Anordnung umfasst eine sterile Endoskophülle nach oben beschriebener Art oder nach einer vorteilhaften Weiterbildung und ein nicht steriles Endoskop. Das Endoskop umfasst einen Endoskopschaft und ein an einem distalen Ende des Endoskopschafts angeordnetes optischen Element. Das optische Element des Endoskops ist für elektromagnetische Strahlung in dem Absorptionswellenlängenbereich transparent. Das Endoskop ist in der Endoskophülle aufgenommen und durch diese gegenüber der Umgebung steril abgeschirmt.

Die Verwendung des nicht sterilen Endoskops in Verbindung mit der sterilen Endoskophülle ist eine kostengünstige Alternative zur Wiedersterilisierung von mehrfach benutzbaren Endoskopen oder zur Verwendung von Einmalendoskopen. Die erfindungsgemäße Endoskophülle gestattet es ferner die Temperatur des optischen Elements zuverlässig zu ermitteln.

Das Endoskop hat ein erstes Sensorelement, das eine von dem optischen Element der Endoskophülle ausgehende elektromagnetische Strahlung in dem Absorptionswellenlängenbereich erfasst. Vorzugsweise ist das Sensorelement derart ausgebildet, die von dem optischen Element ausgehende elektromagnetische Strahlung in dem Absorptionswellenlängenbereich spektral aufgelöst zu erfassen. Aus der durch das erste Sensorelement erfassten elektromagnetischen Strahlung kann die Temperatur des optischen Elements ermittelt werden.

Vorzugsweise hat die Anordnung eine Steuereinheit, die auf Grundlage der durch das erste Sensorelement erfassten elektromagnetischen Strahlung eine Temperatur des optischen Elements der Endoskophülle ermittelt. Die durch die Steuereinheit ermittelte Temperatur kann beispielsweise durch eine Ausgabeeinheit ausgegeben werden. Dadurch kann die Temperatur automatisch überwachen und das Endoskop aus dem Körper des Patienten entfernt oder die Beleuchtung ausgeschaltet werden, bevor die Temperatur des optischen Elements einen Wert erreicht, bei dem es zu einer Gefährdung des Patienten kommen kann.

Es ist vorteilhaft, wenn die Anordnung eine Ausgabeeinheit hat, die ein akustisches und/oder optisches Warnsignal ausgibt, wenn die ermittelte Temperatur des optischen Elements der Endoskophülle einen voreingestellten Wert erreicht und/oder überschreitet. Ein Chirurg muss hierdurch die Temperatur des optischen Elements nicht selbst überwachen und kann sich auf die Durchführung des medizinischen Eingriffs am Patienten konzentrieren. Der voreingestellte Wert liegt vorzugsweise unterhalb einer Temperatur bei der es zu einer Koagulation bzw. Nekrose von Gewebe kommt. Das Endoskop kann so rechtzeitig aus dem Körper des Patienten entfernt oder das Beleuchtungslicht ausgeschaltet werden, bevor es zu einer Gefährdung des Patienten kommt.

Der Endoskopschaft enthält einen ersten Lichtwellenleiter, der mit dem ersten Sensorelement optisch verbunden ist und der in das distale Ende des Endoskopschafts einfallende elektromagnetische Strahlung in dem Absorptionswellenlängenbereich zu dem ersten Sensorelement leitet. Durch den Lichtwellenleiter ist es möglich, das erste Sensorelement in einem proximalen Teil des Endoskops, beispielsweise einem Handstück, anzuordnen. Hierdurch wird ein kompakter Aufbau des Endoskops ermöglicht.

Ferner hat das Endoskop ein zweites Sensorelement, das elektromagnetische Strahlung in dem Absorptionswellenlängenbereich erfasst, und der Endoskopschaft einen zweiten Lichtwellenleiter enthält, der mit dem zweiten Sensorelement optisch verbunden ist und der elektromagnetische Strahlung in dem Absorptionswellenlängenbereich von dem distalen Ende des Endoskopschafts zu dem zweiten Sensorelement leitet. Der zweite Lichtwellenleiter ist an einem distalen Ende optisch verschlossen. Die einzige von dem zweiten Lichtwellenleiter geleite Strahlung ist die von dem Verschluss des zweiten Lichtwellenleiters ausgehende Wärmestrahlung. Hierdurch ist ein Referenzkanal gebildet, mit der die Temperatur des Endoskops, insbesondere des distalen Endes des Endoskops, ermittelt werden kann. Eine Erwärmung des optischen Elements der Endoskophülle verursacht durch Wärmeleitung eine Erwärmung des distalen Endes des Endoskops. Da der Prozess der Wärmeleitung Zeit benötigt, kann mithilfe des Referenzkanals unterschieden werden, ob eine festgestellte Erwärmung des optischen Elements nur kurzfristig erfolgt, beispielsweise durch einen Kontakt des optischen Elements mit einem Laserskalpell, oder langfristig erfolgt, beispielsweise durch eine Verschmutzung des optischen Elements und eine dadurch bedingte Absorption von Beleuchtungslicht.

Es ist vorteilhaft, wenn die Steuereinheit auf Grundlage der durch das erste Sensorelement erfassten elektromagnetischen Strahlung eine Temperatur des optischen Elements der Endoskophülle ermittelt und auf Grundlage der durch das zweite Sensorelement erfassten elektromagnetischen Strahlung eine Temperatur des optischen Elements des Endoskops ermittelt. Vorzugsweise gibt eine Ausgabeeinheit ein akustisches und/oder optisches Warnsignal aus, wenn die ermittelte Temperatur des optischen Elements der Endoskophülle und die ermittelte Temperatur des optischen Elements des Endoskops einen jeweils voreingestellten Wert erreichen und/oder überschreiten. Dadurch wird verhindert, dass es zu Fehlwarnungen kommt, wenn sich das optische Element nur kurzfristig erwärmt, beispielsweise durch den Kontakt mit einem anderen chirurgischen Instrument. Die Ermittlung einer für einen Patienten kritischen Temperatur des optischen Elements wird hierdurch noch zuverlässiger.

Alternativ ermittelt die Steuereinheit eine Differenz zwischen der ermittelten Temperatur des optischen Elements der Endoskophülle und der ermittelten Temperatur des optischen Elements des Endoskops. Vorzugsweise gibt die Ausgabeeinheit ein akustisches oder optisches Warnsignal aus, wenn die ermittelte Temperatur des optischen Elements der Endoskophülle einen voreingestellten Wert erreicht und/oder überschreitet und wenn die ermittelte Differenz zwischen der ermittelten Temperatur des optischen Elements der Endoskophülle und der ermittelten Temperatur des optischen Elements des Endoskops einen voreingestellten Wert erreicht und/oder unterschreitet.

Weiterhin ist vorteilhaft, wenn das optische Element der Endoskophülle wenigstens einen Bereich aufweist, der für elektromagnetische Strahlung in dem Absorptionswellenlängenbereich transparent ist. Vorzugsweise hat das Endoskop ein drittes Sensorelement. Der Endoskopschaft enthält vorzugsweise einen dritten Lichtwellenleiter, der mit dem dritten Sensorelement optisch verbunden ist. Ein distales Ende des dritten Lichtwellenleiters ist dem wenigstens einen Bereich gegenüberliegend angeordnet. Der dritte Lichtwellenleiter leitet in das distale Ende des dritten Lichtwellenleiters einfallende elektromagnetische Strahlung in dem Absorptionswellenlängenbereich zu dem dritten Sensorelement. Elektromagnetische Strahlung in dem Absorptionswellenlängenbereich, die beispielsweise von Quellen im Situs emittiert wird, kann das optische Element in dem wenigstens einen Bereich ungehindert passieren und wird durch den dritten Lichtwellenleiter zu dem dritten Sensorelement geleitet und durch dieses erfasst. Da aus dem Spektrum dieser Strahlung die Temperatur der Quelle im Situs ermittelt werden kann, ist es möglich, eine Temperaturmessung im Situs, beispielsweise zu diagnostischen Zwecken, durchzuführen. Das dritte Sensorelement kann ein Bildsensor sein. Hierdurch kann die durch das dritte Sensorelement erfasste elektromagnetische Strahlung in dem Absorptionswellenlängenbereich zur Bildgebung verwendet werden.

Bei dem Vorsehen von unterschiedlichen optischen Elementen in verschiedenen Endoskophüllen kann über die Wahl der Endoskophülle die Abbildung des mit Hilfe des Endoskops detektierten Lichts verändert werden, sodass die optischen Bildaufnahmeeigenschaften des Endoskops durch die Wahl der Hülle verändert werden können.

Bei dem Endoskop kann es sich um ein Monoendoskop, d.h. ein Endoskop mit nur einem optischen Kanal, oder ein stereoskopisches Endoskop handeln.

Weitere Merkmale und Vorteile der Erfindung ergeben sich aus der folgenden Beschreibung, die die Erfindung anhand von Ausführungsbeispielen im Zusammenhang mit den beigefügten Figuren näher erläutert.

Die folgenden Ausführungsbeispiele 1-5 und 7 sind nicht gemäß der Erfindung. Es zeigen:
- Figur 1: eine Anordnung zur sterilen Handhabung eines nicht sterilen Endoskops in einer sterilen Umgebung mit einer sterilen Endoskophülle gemäß einem ersten Ausführungsbeispiel;
- Figur 2a: eine schematische Schnittdarstellung einer Anordnung mit einem Monoendoskop gemäß einem zweiten Ausführungsbeispiel;
- Figur 2b: eine schematische Darstellung des Monoendoskops nach Figur 2a von einer distalen Seite her gesehen;
- Figur 3a: eine schematische Schnittdarstellung einer Anordnung mit einem Monoendoskop gemäß einem dritten Ausführungsbeispiel;
- Figur 3b: eine schematische Darstellung des Monoendoskops nach Figur 3a von einer distalen Seite hergesehen;
- Figur 4a: eine schematische Schnittdarstellung einer Anordnung mit einem Monoendoskop gemäß einem vierten Ausführungsbeispiel;
- Figur 4b: eine schematische Darstellung des Monoendoskops nach Figur 4a von einer distalen Seite her gesehen;
- Figur 5a: eine schematische Schnittdarstellung einer Anordnung mit einem stereoskopischen Endoskop gemäß einem fünften Ausführungsbeispiel;
- Figur 5b: eine schematische Darstellung des stereoskopischen Endoskops gemäß nach Figur 5a von einer distalen Seite her gesehen;
- Figur 6a: eine schematische Schnittdarstellung einer Anordnung mit einem stereoskopischen Endoskop gemäß einem sechsten Ausführungsbeispiel gemäß der Erfindung;
- Figur 6b: eine schematische Darstellung des stereoskopischen Endoskops nach Figur 6a von einer distalen Seite her gesehen;
- Figur 7a: eine schematische Schnittdarstellung einer Anordnung mit einem stereoskopischen Endoskop gemäß einem siebenten Ausführungsbeispiel;
- Figur 7b: eine schematische Darstellung des stereoskopischen Endoskops nach Figur 7a von einer distalen Seite her gesehen in einer schematischen Darstellung; und
- Figur 7c: eine schematische Darstellung eines optischen Elements der Endoskophülle nach den Figur 7a und 7b von einer proximalen Seite her gesehen;

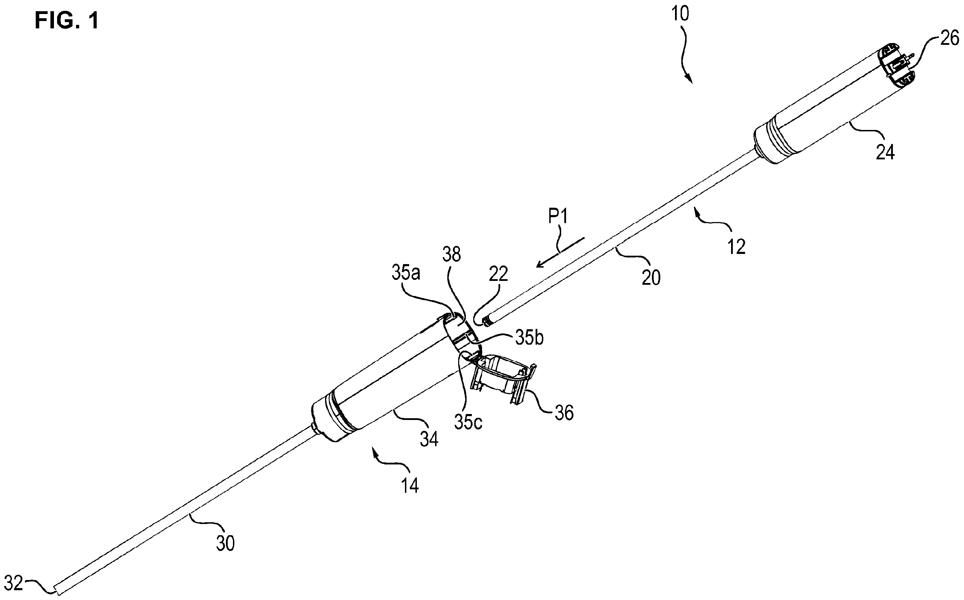

Figur 1 zeigt perspektivische Darstellung einer Anordnung 10 zur sterilen Handhabung eines nicht sterilen Endoskops 12 in einer sterilen Umgebung gemäß einem ersten nicht-erfindungsgemäßen

Ausführungsbeispiel. Die Anordnung 10 umfasst neben dem Endoskop 12 eine sterile Endoskophülle 14.

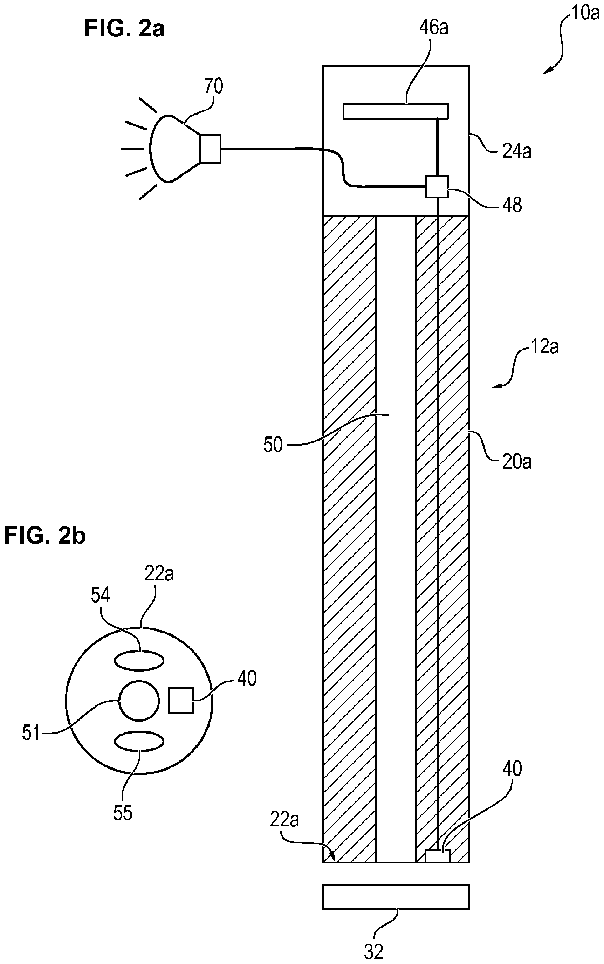

Das Endoskop 12 hat einen an einem proximalen Ende eines Endoskopschafts 20 angeordneten Endoskopkörper 24. Das Endoskops 12, insbesondere der innere Aufbau des Endoskops 12, wird nachfolgend anhand der Figuren 2a, 2b, 3a, 3b, 4a, 4b, 5a, 5b, 6a, 6b, 7a und 7b näher beschrieben.

Die Endoskophülle 14 umfasst einen vorderen Teil 30 zur Aufnahme des zumindest teilweise in einen Körper eines Patienten einführbaren Endoskopschafts 20. Der vordere Teil 30 der Endoskophülle 14 ist an einem distalen Ende mit Hilfe eines optischen Elements 32 verschlossen, das elektromagnetische Strahlung in einem Absorptionswellenlängenbereich von 8 µm bis 14 µm absorbiert und in einem optischen Wellenlängenbereich von 380 nm bis 780 nm transparent ist. Da das optische Element 32 die elektromagnetische Strahlung in dem Absorptionswellenlängenbereich absorbiert, hat es einen sehr geringen Reflexionsgrad und einen sehr hohen Emissionsgrad in dem Absorptionswellenlängenbereich. Damit entspricht die von dem optischen Element 32 ausgehende elektromagnetische Strahlung in dem Absorptionswellenlängenbereich im Wesentlichen der von dem optischen Element 32 ausgehenden Wärmestrahlung, die lediglich von der Temperatur des optischen Elements 32 abhängig ist. Aus dem Spektrum dieser Strahlung kann die Temperatur des optischen Elements 32 ermittelt werden.

Die Endoskophülle 14 umfasst ferner ein Mittelteil 34 zur Aufnahme des Endoskopkörpers 24 und ein mit dem Mittelteil 34 verbundenes Verschlusselement 28 mit einer Sterilschleuse. Durch das Verschlusselement 36 ist eine Zuführ- und Entnahmeöffnung 38 der Endoskophülle 14 zum Einführen und Herausnehmen das Endoskops 12 in bzw. aus der Endoskophülle 14 gebildet. Mit Hilfe der Sterilschleuse wird ein Kontaktbereich 26 des Endoskops 12 mit elektrischen Kontakt und optischen Verbindungselementen steril abgeschirmt.

Zur Aufnahme des Endoskops 12 in der Endoskophülle 14 wird das Endoskop 12 in Richtung des Pfeils P1 durch die geöffnete Zuführ- und Entnahmeöffnung 38 in die Endoskophülle 14 eingeführt. Hierzu wird der Endoskopschaft 20 zuerst in die Zuführ- und Entnahmeöffnung 38 eingeführt und nachfolgend bis in den vorderen Teil 30 der Endoskophülle 14 geschoben, so dass eine Spitze 22 des Endoskopschafts 20 dem an dem distalen Ende des vorderen Teils 30 angeordneten optischen Element 32 der Endoskophülle 14 gegenüberliegend angeordnet ist. Beim Einführen eines Endoskopkörpers 24 durch die Zuführ- und Entnahmeöffnung 38 in den Mittelteil 34 der Endoskophülle 14 wird der Endoskopkörper 24 durch innen im Mittelteil 34 der Endoskophülle 14 vorhandene Führungsstege 35a bis 35c geführt und in einer vorbestimmten Lage im Mittelteil 34 der Endoskophülle 14 gehalten.

Figur 2a zeigt eine schematische Schnittdarstellung einer Anordnung 10a gemäß einem zweiten nicht-erfindungsgemäßen

Ausführungsbeispiel. Gleiche Elemente oder Elemente mit gleicher Funktion haben dieselben Bezugszeichen. Die Anordnung 10a umfasst ein Monoendoskop 12a, das ein erstes Sensorelement 40 hat, eine Endoskophülle 14 nach Figur 1, von der in Figur 2a nur das optische Element 32 dargestellt ist, und eine Ausgabeeinheit 70. Das nicht sterile Monoendoskop 12a ist durch die sterile Endoskophülle 14 aufgenommen und dadurch gegenüber der Umgebung steril abgeschirmt.

Das Monoendoskop 12a hat einen in distaler Richtung von einem Endoskopkörper 24a abstehenden Endoskopschaft 20a, an dessen distaler Spitze 22a das erste Sensorelement 40 angeordnet ist. Der Endoskopschaft 20a umfasst ferner eine erste Beobachtungsoptik 50. Der Endoskopkörper 24a schließt sich am proximalen Ende des Endoskopschafts 20a an diesen an. In dem Endoskopkörper 24a sind insbesondere eine Steuereinheit 48 und ein Bildsensor 46 angeordnet.

Die von dem optischen Element 32 ausgehende elektromagnetische Strahlung in dem Absorptionswellenlängenbereich ist im Wesentlichen die Wärmestrahlung des optischen Elements 32, die nur von dessen Temperatur abhängig ist. Diese Strahlung wird durch das an der distalen Spitze 22a des Endoskopschafts 20a angeordnete erste Sensorelement 40 erfasst. Aus dem Spektrum der erfassten Strahlung ermittelt die Steuereinheit 48 die Temperatur des optischen Elements 32. Übersteigt die durch die Steuereinheit 48 ermittelte Temperatur des optischen Elements 32 einen voreingestellten Grenzwert, steuert die Steuereinheit 48 die Ausgabeeinheit 70 derart, dass diese ein optisches und/oder akustisches Warnsignal ausgibt.

Die erste Beobachtungsoptik 50 bildet einen optischen Kanal, der in die Spitze 22a des Endoskopschafts 20a einfallendes Beobachtungslicht in einem optischen Wellenlängenbereich von dem distalen Ende des Endoskopschafts 20a zu dem proximalen Ende des Endoskopschafts 20a leitet. Nach Durchtritt durch die erste Beobachtungsoptik 50 fällt das Beobachtungslicht auf den Bildsensor 46 und wird durch diesen in ein Signal bzw. Daten gewandelt. Das Signal wird bzw. die Datenwerden durch die Steuereinheit 48 zur Bildanzeige weiterverarbeitet. Hierdurch ist eine Beobachtung eines Bereichs distal der Spitze 22a möglich. Alternativ kann das Signal bzw. können die Daten auch durch eine weitere Steuereinheit außerhalb des Endoskops 12a zur Bildanzeige weiterverarbeitet werden.

Figur 2b zeigt eine schematische Darstellung des Monoendoskops 12a nach Figur 2a von einer distalen Seite her gesehen, d.h. die Spitze 22a des Monoendoskops 12a. Das distale Ende 51 der ersten Beobachtungsoptik 50 ist mittig der Spitze 22a angeordnet. In der Darstellung der Figur 2b sind oberhalb und unterhalb des distalen Endes 51 der ersten Beobachtungsoptik 50 je ein distales Ende 54, 55 einer Beleuchtungsoptik zum Beleuchten des Bereichs distal der Spitze 22a angeordnet. Das erste Sensorelement 40 ist in der Darstellung von Figur 2b rechts des distalen Endes 51 der Beobachtungsoptik 50 angeordnet.

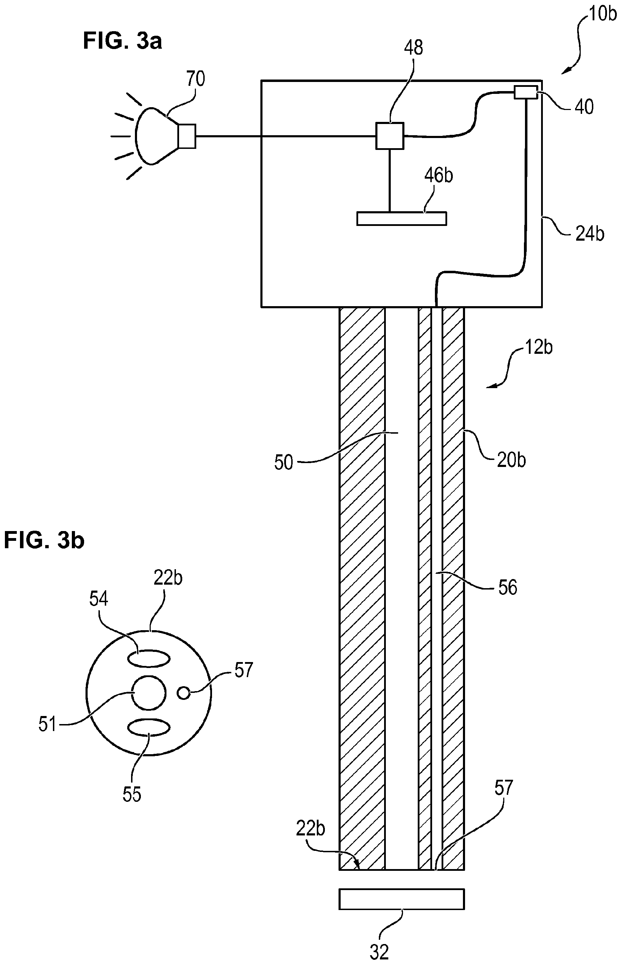

Figur 3a zeigt eine schematische Schnittdarstellung einer Anordnung 10b gemäß einem dritten nicht-erfindungsgemäßen Ausführungsbeispiel. Die Anordnung 10b umfasst ein Monoendoskop 12b, das das erste Sensorelement 40 hat. Die Anordnung 10b umfasst ferner einen ersten Lichtwellenleiter 56, der mit dem ersten Sensorelement 40 optisch verbunden ist. Weiterhin umfasst die Anordnung 10b die Endoskophülle 14 nach

Figur 1, von der in Figur 2a nur das optische Element 32 gezeigt ist, und die Ausgabeeinheit 70. Das nicht sterile Monoendoskop 12b ist in der sterilen Endoskophülle 14 aufgenommen und durch diese gegenüber der Umgebung steril abgeschirmt. Die Anordnung 10b gemäß dem dritten Ausführungsbeispiel nach Figur 3a unterscheidet sich von der Anordnung 10a gemäß dem zweiten Ausführungsbeispiel nach Figur 2a im Wesentlichen durch eines ersten Lichtwellenleiters 56.

Der Endoskopschaft 20b des Monoendoskops 12b umfasst die erste Beobachtungsoptik 50 und den ersten Lichtwellenleiter 56. Das Monoendoskop 12b hat ferner einen an dem proximalen Ende des Endoskopschafts 20b angeordneten Endoskopkörper 24b, in dem insbesondere das erste Sensorelement 40, die Steuereinheit 48 und der Bildsensor 46 angeordnet sind.

Die von dem optischen Element 32 ausgehende elektromagnetische Strahlung in dem Absorptionswellenlängenbereich fällt in das an der distalen Spitze 22b des Endoskopschafts 20a angeordnete distale Ende 57 des ersten Lichtwellenleiters 56. Der erste Lichtwellenleiter 56 leitet die elektromagnetische Strahlung in dem Absorptionswellenlängenbereich von seinem distalen Ende 57 bis zu dem ersten Sensorelement 40, das diese Strahlung erfasst. Aus dem Spektrum der erfassten Strahlung ermittelt die Steuereinheit 48 die Temperatur des optischen Elements 32.

Übersteigt die durch die Steuereinheit 48 ermittelte Temperatur des optischen Elements 32 einen voreingestellten Grenzwert, steuert die Steuereinheit 48 die Ausgabeeinheit 70 derart, dass diese ein optisches und/oder akustisches Warnsignal ausgibt.

Figur 3b zeigt eine schematische Darstellung des Monoendoskops 12b nach Figur 3a von einer distalen Seite her gesehen. Figur 3a zeigt insbesondere die Spitze 22b des Monoendoskops 12b. In der Darstellung von Figur 3b ist das distale Ende 57 des ersten Lichtwellenleiters 56 rechts des distalen Endes 51 der Beobachtungsoptik 50 angeordnet.

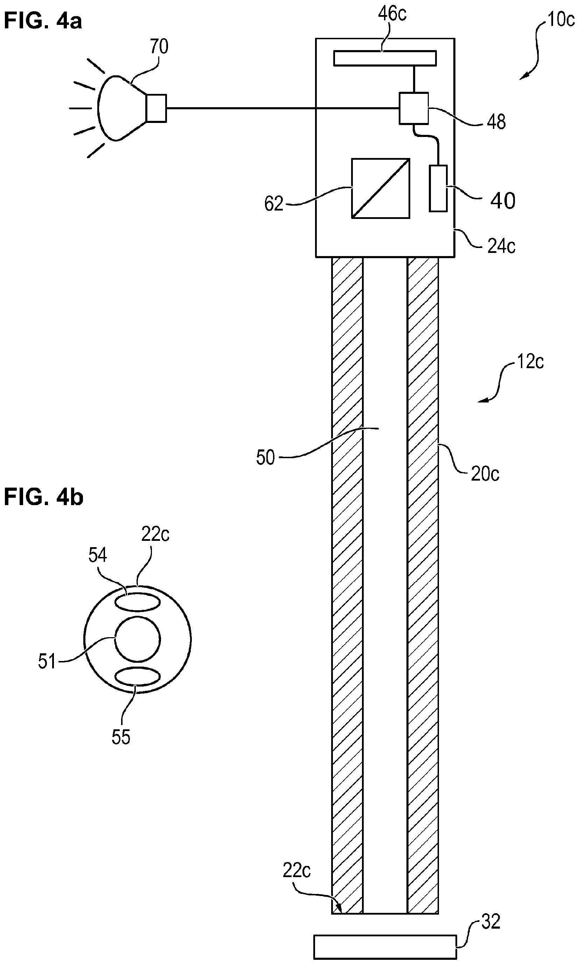

Figur 4a zeigt eine schematische Schnittdarstellung einer Anordnung 10c gemäß einem vierten nicht-erfindungsgemäßen Ausführungsbeispiel. Die Anordnung umfasst ein Monoendoskop 12c, welches das erste Sensorelement 40 und einen Strahlteiler 62 hat, eine Endoskophülle 14 nach Figur 1, von der in Figur 3a nur das optische Element 32 gezeigt ist, sowie die Ausgabeeinheit 70. Das nicht sterile Monoendoskop 12c ist durch die sterile Endoskophülle 14 aufgenommen und durch diese gegenüber der Umgebung steril abgeschirmt. Die Anordnung 10c gemäß dem vierten Ausführungsbeispiel nach Figur 3a unterscheidet sich von der Anordnung 10a gemäß dem zweiten Ausführungsbeispiel nach Figur 2a im Wesentlichen durch das Vorsehen eines Strahlteilers 62.

Der Endoskopschaft 20c enthält die erste Beobachtungsoptik 50. Das Monoendoskop 12c hat ferner einen an dem proximalen Ende des Endoskopschafts 20c angeordneten Endoskopkörper 24c, in dem insbesondere das erste Sensorelement 40, die Steuereinheit 48 und der Strahlteiler 62 angeordnet sind.

Die von dem optischen Element 32 ausgehende elektromagnetische Strahlung in dem Absorptionswellenlängenbereich fällt auf den an einer distalen Spitze 22c des Endoskopschafts 20c angeordnete distale Ende 51 der ersten Beobachtungsoptik 50. Die erste Beobachtungsoptik 50 leitet die elektromagnetische Strahlung in dem Absorptionswellenlängenbereich von ihrem distalen Ende 51 bis zu Strahlteiler 62. Der Strahlteiler 62 lenkt die elektromagnetische Strahlung in dem Absorptionswellenlängenbereich auf das erste Sensorelement 40, das diese Strahlung erfasst. Aus dem Spektrum der erfassten Strahlung ermittelt die Steuereinheit 48 die Temperatur des optischen Elements 32. Übersteigt die durch die Steuereinheit 48 ermittelte Temperatur des optischen Elements 32 einen voreingestellten Grenzwert, steuert die Steuereinheit 48 die Ausgabeeinheit 70 derart, dass diese ein optisches und/oder akustisches Warnsignal ausgibt.

Figur 4b zeigt eine schematische Darstellung des Monoendoskop 12c nach Figur 4a von einer distalen Seite her gesehen. Das distale Ende 51 der ersten Beobachtungsoptik 50 ist mittig angeordnet. In sind oberhalb und unterhalb des distalen Endes 51 der ersten Beobachtungsoptik 50 je ein distales Ende 54, 55 einer Beleuchtungsoptik zum Beleuchten des Bereichs distal der Spitze 22c dargestellt.

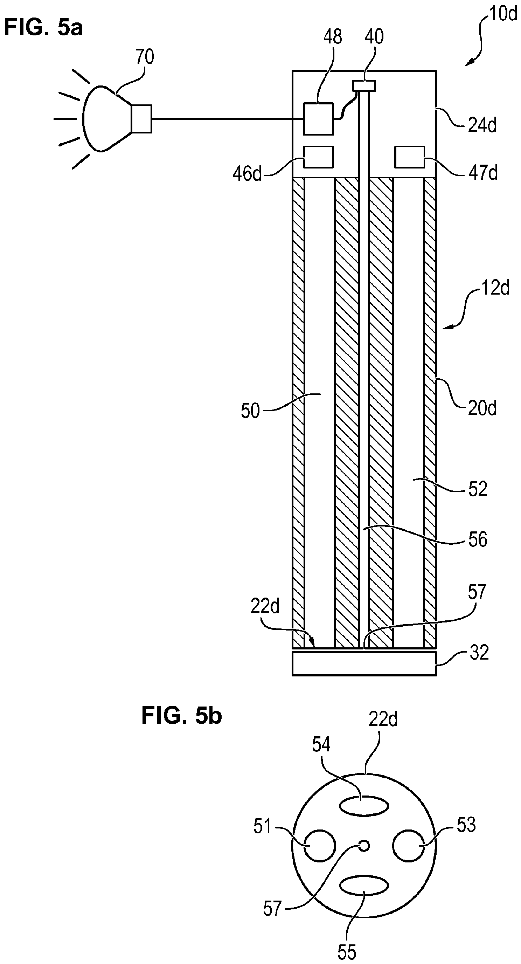

Figur 5a zeigt eine schematische Schnittdarstellung einer Anordnung 10d gemäß einem fünften nicht-erfindungsgemäßen Ausführungsbeispiel. Die Anordnung 10d umfasst ein stereoskopisches Endoskop 12d, das das erste Sensorelement 40 und den ersten Lichtwellenleiter 56 hat. Die Anordnung 10d umfasst ferner die Endoskophülle 14 nach Figur 1, von der in Figur 5a nur das optische Element 32 gezeigt ist und die Ausgabeeinheit 70. Das nicht sterile Endoskop 12d ist in der sterilen Endoskophülle 14 aufgenommen und durch diese gegenüber der Umgebung steril abgeschirmt.

Der Endoskopschaft 20d umfasst die erste Beobachtungsoptik 50, eine zweite Beobachtungsoptik 52 und den ersten Lichtwellenleiter 56. Das Endoskop 12d hat ferner einen an dem proximalen Ende des Endoskopschafts 20b angeordneten Endoskopkörper 24d, in dem insbesondere das erste Sensorelement 40 und die Steuereinheit 48 angeordnet sind.

Die von dem optischen Element 32 ausgehende elektromagnetische Strahlung in dem Absorptionswellenlängenbereich fällt in das an einer distalen Spitze 22d des Endoskopschafts 20d angeordnete distale Ende 57 des ersten Lichtwellenleiters 56. Der erste Lichtwellenleiter 56 leitet die elektromagnetische Strahlung in dem Absorptionswellenlängenbereich von seinem distalen Ende 57 bis zu dem ersten Sensorelement 40, das diese Strahlung erfasst. Aus dem Spektrum der erfassten Strahlung ermittelt die Steuereinheit 48 die Temperatur des optischen Elements 32. Übersteigt die durch die Steuereinheit 48 ermittelte Temperatur des optischen Elements 32 einen voreingestellten Grenzwert, steuert die Steuereinheit 48 die Ausgabeeinheit 70 derart, dass diese ein optisches und/oder akustisches Warnsignal ausgibt.

Die erste Beobachtungsoptik 50 und die zweite Beobachtungsoptik 52 bilden jeweils einen optischen Kanal, der in die Spitze 22d des Endoskopschafts 20d einfallendes Beobachtungslicht in einem optischen Wellenlängenbereich von dem distalen Ende des Endoskopschafts 20d zu dem proximalen Ende des Endoskopschafts 20d leitet. Hierdurch ist eine stereoskopische Beobachtung eines Bereichs distal der Spitze 22d möglich.

Figur 5b zeigt eine schematische Schnittdarstellung des stereoskopischen Endoskops 12d nach Figur 5a von einer distalen Seite her gesehen Das distale Ende 57 des ersten Lichtwellenleiters 56 ist mittig der Spitze 22d des Endoskops 12d angeordnet. In der Darstellung der Figur 5b sind oberhalb und unterhalb des distalen Endes 57 des ersten Lichtwellenleiter 56 je ein distales Ende 54, 55 der Beleuchtungsoptik zum Beleuchten des Bereichs distal der Spitze 22d angeordnet. Links des distalen Endes 57 des ersten Lichtwellenleiters 56 ist ein distales Ende 51 der ersten Beobachtungsoptik 50 angeordnet. Rechts des distalen Endes 57 des ersten Lichtwellenleiters 56 ist ein distales Ende 53 der zweiten Beobachtungsoptik 53 angeordnet.

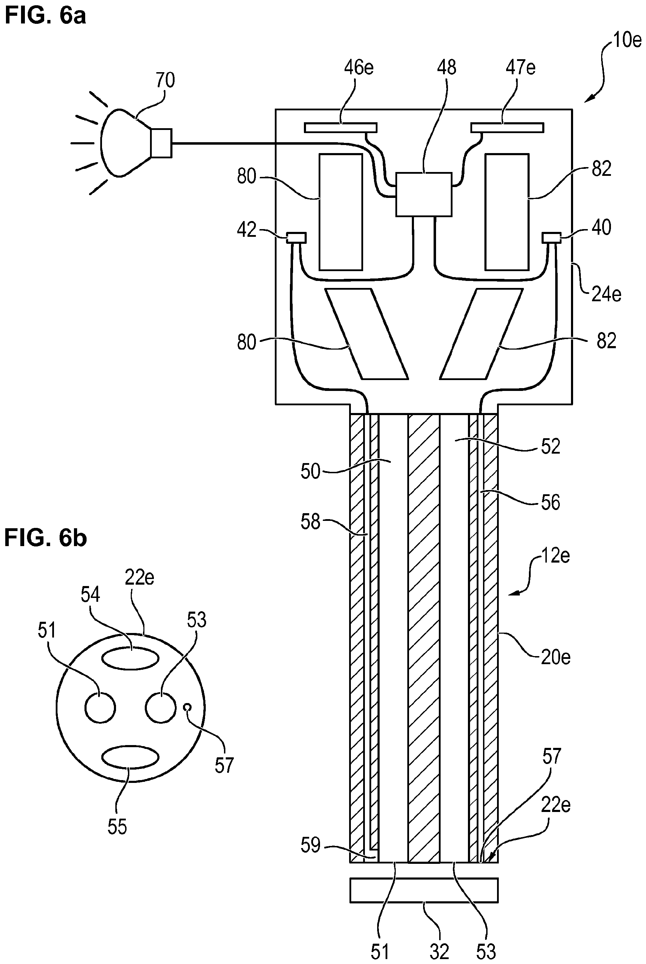

Figur 6a zeigt eine schematische Schnittdarstellung einer Anordnung 10e gemäß einem sechsten Ausführungsbeispiel. Die Anordnung 10e umfasst ein stereoskopisches Endoskop 12e, das das erste Sensorelement 40, ein zweites Sensorelement 42, den ersten Lichtwellenleiter 56 und einen zweiten Lichtwellenleiter 58 hat. Die Anordnung 10e umfasst ferner die Endoskophülle 14 nach Figur 1, von der in Figur 6a nur das optische Element 32 dargestellt ist und die Ausgabeeinheit 70. Das nicht sterile Endoskop 12e ist in der sterilen Endoskophülle 14 aufgenommen und durch diese gegenüber der Umgebung steril abgeschirmt. Die Anordnung 10e gemäß dem sechsten Ausführungsbeispiel nach Figur 6a unterscheidet sich von der Anordnung 10d gemäß dem vierten Ausführungsbeispiel nach Figur 4a im Wesentlichen durch Vorsehen eines zweiten Lichtwellenleiters 58 und eines zweiten Sensorelements 42.

Der Endoskopschaft 20e des Endoskops 12e umfasst die erste Beobachtungsoptik 50, die zweite Beobachtungsoptik 52, den ersten Lichtwellenleiter 56, der mit dem ersten Sensorelement optisch verbunden ist. Der Endoskopschaft 20e umfasst ferner den zweiten Lichtwellenleiter 58, der mit dem zweiten Sensorelement 42 optisch verbunden ist und der an seinem distalen Ende 59 optisch verschlossen ist. Das Endoskop 12e hat ferner einen an dem proximalen Ende des Endoskopschafts 20e angeordneten Endoskopkörper 24e, in dem insbesondere das erste Sensorelement 40, das zweite Sensorelement 42, ein erster Bildsensor 46e, ein zweiter Bildsensor 47e, die Steuereinheit 48, weitere optische Elemente, wie beispielsweise Prismen, Linsen oder Blenden, die der ersten Beobachtungsoptik 50 zugeordnet sind und die allgemein mit dem Bezugszeichen 80 versehen sind, und weitere optische Elemente, die der zweiten Beobachtungsoptik 52 zugeordnet sind und die allgemein mit dem Bezugszeichen 82 versehen sind, angeordnet sind.

Die von dem optischen Element 32 ausgehende elektromagnetische Strahlung in dem Absorptionswellenlängenbereich fällt in das an einer distalen Spitze 22e des Endoskopschafts 20e angeordnete distale Ende 57 des ersten Lichtwellenleiters 56. Der erste Lichtwellenleiter 56 leitet die elektromagnetische Strahlung in dem Absorptionswellenlängenbereich von seinem distalen Ende 57 bis zu dem ersten Sensorelement 40, das diese Strahlung erfasst. Aus dem Spektrum der erfassten Strahlung ermittelt die Steuereinheit 48 die Temperatur des optischen Elements 32.

Da der zweite Lichtwellenleiter 58 an seinem distalen Ende 59 optisch verschlossen ist, ist die einzige von dem zweiten Lichtwellenleiter 58 geleite Strahlung die von dem Verschluss des zweiten Lichtwellenleiters 58 selbst ausgehende Wärmestrahlung. Diese wird durch das zweite Sensorelement 42 erfasst. Aus dem Spektrum der erfassten Strahlung ermittelt die Steuereinheit 48 die Temperatur des distalen Endes des Endoskops 12e. Eine Erwärmung des optischen Elements 32 der Endoskophülle 14 verursacht durch Wärmeleitung eine Erwärmung des distalen Endes des Endoskops 12e. Da der Prozess der Wärmeleitung Zeit benötigt, kann unterschieden werden, ob eine festgestellte Erwärmung des optischen Elements 32 nur kurzfristig erfolgt, beispielsweise durch einen Kontakt des optischen Elements 32 mit einem chirurgischen Instrument, wie beispielsweise einem Laserskalpell, oder langfristig erfolgt, beispielsweise durch eine Verschmutzung des optischen Elements 32 und eine dadurch bedingte Absorption von Beleuchtungslicht.

Die Ausgabeeinheit 70 gibt ein akustisches und/oder optisches Warnsignal aus, wenn die ermittelte Temperatur des optischen Elements 32 der Endoskophülle 14e und die ermittelte Temperatur des optischen Elements des Endoskops 12e einen jeweils voreingestellten Wert überschreiten.

Die erste Beobachtungsoptik 50 und die zweite Beobachtungsoptik 52 bilden jeweils einen optischen Kanal, der in die Spitze 22e des Endoskopschafts 20e einfallendes Beobachtungslicht in dem optischen Wellenlängenbereich von dem distalen Ende des Endoskopschafts 20e zu dem proximalen Ende des Endoskopschafts 20e leitet, wo es in die weiteren optischen Elemente 80, 82 eintritt. Das von der ersten Beobachtungsoptik 50 geleitete Beobachtungslicht fällt nach Durchtritt durch die weiteren optischen Elemente 80 auf den ersten Bildsensor 46e. Das von der zweiten Beobachtungsoptik 52 geleitete Beobachtungslicht fällt nach Durchtritt durch die weiteren optischen Elemente 82 auf den zweiten Bildsensor 47e. Der erste und der zweite Bildsensor 46e, 47e wandeln das erfasste Beobachtungslicht jeweils in ein Signal oder in Daten. Die Signale bzw. Daten werden durch die Steuereinheit 48 zur Bildanzeige weiterverarbeitet. Hierdurch ist eine Beobachtung eines Bereichs distal der Spitze 22e möglich. Alternativ können die Signale bzw. Daten auch durch eine weitere Steuereinheit außerhalb des Endoskops 12e zur Bildanzeige weiterverarbeitet werden. Hierdurch ist eine stereoskopische Beobachtung eines Bereichs distal der Spitze 22e möglich.

Figur 6b zeigt eine schematische Darstellung des stereoskopischen Endoskops 12e nach Figur 6a von einer distalen Seite her gesehen In Figur 6b ist das distales Ende 51 links der ersten Beobachtungsoptik 50dargestellt. Rechts der Mitte der Spitze 22e das distales Ende 53 der zweiten Beobachtungsoptik 53 angeordnet. Das distale Ende 57 des ersten Lichtwellenleiters 56 ist rechts des distalen Endes 53 der zweiten Beobachtungsoptik 53 angeordnet. Oberhalb und unterhalb des distalen Endes 51 der ersten Beobachtungsoptik 50 und des distalen Endes 53 der zweiten Beobachtungsoptik 52 sind je ein distales Ende 54, 55 einer Beleuchtungsoptik zum Beleuchten des Bereichs distal der Spitze 22e angeordnet.

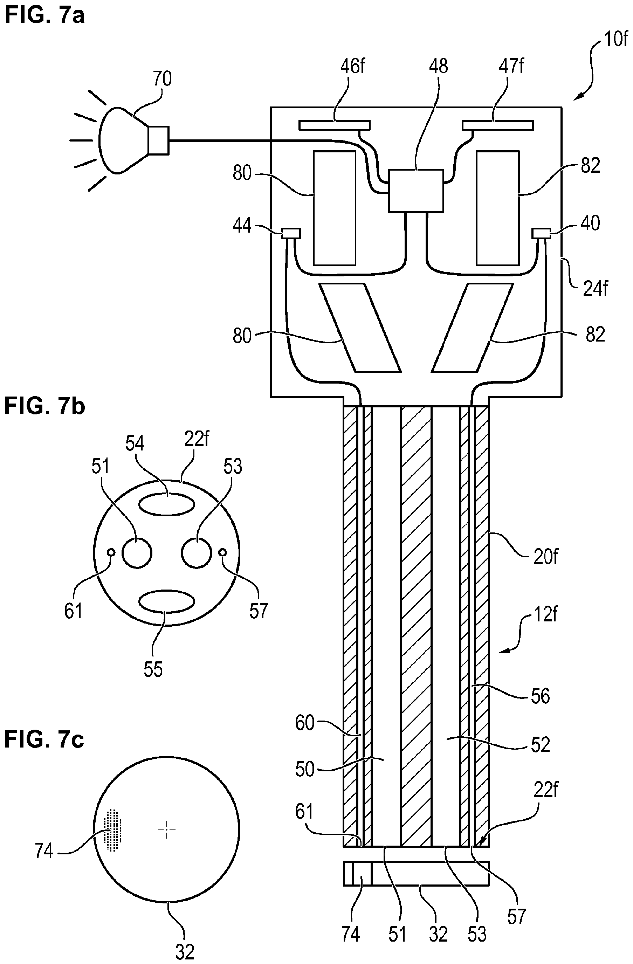

Figur 7a zeigt eine schematische Schnittdarstellung einer Anordnung 10f gemäß einem siebenten nicht-erfindungsgemäßen Ausführungsbeispiel. Die Anordnung 10f umfasst ein stereoskopisches Endoskop 12f, das das erste Sensorelement 40, ein drittes Sensorelement 44, den ersten Lichtwellenleiter 56 und einen dritten Lichtwellenleiter 60 hat. Die Anordnung 10f umfasst ferner eine Endoskophülle, von der in Figur 7a nur ein optisches Element 32f dargestellt ist, sowie die die Ausgabeeinheit 70.

Von der Endoskophülle ist in Figur 7a nur ein optisches Element 32f gezeigt, das einen Bereich 74 aufweist, der für elektromagnetische Strahlung in dem Absorptionswellenlängenbereich durchlässig ist. Der übrige Aufbau der Endoskophülle nach Figur 7a entspricht der Endoskophülle 14 nach Figur 1. Das nicht sterile Endoskop 12f ist in der sterilen Endoskophülle aufgenommen und durch diese gegenüber der Umgebung steril abgeschirmt.

Der Endoskopschaft 20f des Endoskops 12f umfasst die erste Beobachtungsoptik 50, die zweite Beobachtungsoptik 52, den ersten Lichtwellenleiter 56 der mit dem ersten Sensorelement optisch verbunden ist, und den dritten Lichtwellenleiter 60, der mit dem dritten Sensorelement 44 optisch verbunden ist. Das Endoskop 12f hat ferner einen an dem proximalen Ende des Endoskopschafts 20f angeordneten Endoskopkörper 24f, in dem insbesondere das erste Sensorelement 40, das dritte Sensorelement 44, ein erster Bildsensor 46f, ein zweiter Bildsensor 47f, die Steuereinheit 48, die weiteren optische Elemente 80, die der ersten Beobachtungsoptik 50 zugeordnet sind, und die weiteren optische Elemente 82, die der zweiten Beobachtungsoptik 52 zugeordnet sind, angeordnet sind.

Die von dem optischen Element 32 ausgehende elektromagnetische Strahlung in dem Absorptionswellenlängenbereich fällt in das an einer distalen Spitze 22f des Endoskopschafts 20f angeordnete distale Ende 57 des ersten Lichtwellenleiters 56. Der erste Lichtwellenleiter leitet die elektromagnetische Strahlung in dem Absorptionswellenlängenbereich von seinem distalen Ende 57 bis zu dem ersten Sensorelement 40, das diese Strahlung erfasst. Aus dem Spektrum der erfassten Strahlung ermittelt die Steuereinheit 48 die Temperatur des optischen Elements 32.

Übersteigt die durch die Steuereinheit 48 ermittelte Temperatur des optischen Elements 32 einen voreingestellten Grenzwert, steuert die Steuereinheit 48 die Ausgabeeinheit 70 derart, dass diese ein optisches und akustisches Warnsignal ausgibt.

Ein distales Ende 61 des dritten Lichtwellenleiters 60 ist dem Bereich 74 des optischen Elements 32, der für elektromagnetische Strahlung in dem Absorptionswellenlängenbereich transparent ist, gegenüberliegend angeordnet. Der dritte Lichtwellenleiter 60 leitet die in sein distales Ende 61 einfallende Strahlung zu dem dritten Sensorelement 44. Aus dem durch das dritte Sensorelement 44 erfassten Spektrum ermittelt die Steuereinheit 48 eine Temperatur. Die elektromagnetische Strahlung in dem Absorptionswellenlängenbereich, die beispielsweise von Quellen im Situs emittiert wird, kann das optische Element 32 in dem Bereich 74 ungehindert passieren. Da aus dem Spektrum dieser Strahlung die Temperatur der Quelle im Situs ermittelt wird, kann eine Temperaturmessung im Situs erfolgen.

Die erste Beobachtungsoptik 50 und die zweite Beobachtungsoptik 52 bilden jeweils einen optischen Kanal, der in die Spitze 22f des Endoskopschafts 20f einfallendes Beobachtungslicht in einem optischen Wellenlängenbereich von dem distalen Ende des Endoskopschafts 20f zu dem proximalen Ende des Endoskopschafts 20f leitet, wo es in die weiteren optischen Elemente 80, 82 eintritt. Das von der ersten Beobachtungsoptik 50 geleitete Beobachtungslicht fällt nach Durchtritt durch die weiteren optischen Elemente 80 auf den ersten Bildsensor 46f. Das von der zweiten Beobachtungsoptik 52 geleitete Beobachtungslicht fällt nach Durchtritt durch die weiteren optischen Elemente 82 auf den zweiten Bildsensor 47f. Der erste und der zweite Bildsensor 46f, 47f wandeln das erfasste Beobachtungslicht jeweils in ein Signal. Die Signale werden durch die Steuereinheit 48 zur Bildanzeige weiterverarbeitet. Hierdurch ist eine Beobachtung eines Bereichs distal der Spitze 22f möglich. Alternativ können die Signale auch durch eine weitere Steuereinheit außerhalb des Endoskops 12f zur Bildanzeige weiterverarbeitet werden. Hierdurch ist eine stereoskopische Beobachtung eines Bereichs distal der Spitze 22f möglich.

Figur 7b zeigt eine schematische Darstellung des stereoskopischen Endoskops 12f nach Figur 7a von einer distalen Seite her gesehen. In Figur 7b ist links der Mitte der Spitze 22f ist das distales Ende 51 der ersten Beobachtungsoptik 50 dargestellt. Rechts der Mitte der Spitze 22f das distales Ende 53 der zweiten Beobachtungsoptik 53 dargestellt. Das distale Ende 57 des ersten Lichtwellenleiters 56 ist rechts des distalen Endes 53 der zweiten Beobachtungsoptik 53 angeordnet. Das distale Ende 61 des dritten Lichtwellenleiters 60 ist links des distalen Endes 51 der ersten Beobachtungsoptik 50 angeordnet. Oberhalb und unterhalb des distalen Endes 51 der ersten Beobachtungsoptik 50 und des distalen Endes 53 der zweiten Beobachtungsoptik 52 sind je ein distales Ende 54, 55 einer Beleuchtungsoptik zum Beleuchten des Bereichs distal der Spitze 22f angeordnet.

Figur 7c zeigt eine schematische Darstellung des optischen Elements 32f der Endoskophülle nach Figur 7a von einer proximalen Seite her gesehen. Das optische Element 32f ist für elektromagnetische Strahlung in dem Absorptionswellenlängenberiech nur in dem Bereich 74 transparent. Der Bereich 74 ist derart auf dem optischen Element 32f angeordnet, dass dieser dem distalen Ende 61 des dritten Lichtwellenleiters 60 gegenüberliegt, wenn das Endoskop 12f bestimmungsgemäß in der Endoskophülle aufgenommen ist.

### Bezugszeichenliste

- 10: Anordnung
- 12: Endoskop
- 14: Endoskophülle
- 20: Endoskopschaft
- 22: Spitze
- 24: Endoskopkörper
- 26: Kontaktbereich
- 30: Vorderer Teil
- 32: Optisches Element
- 34: Mittlerer Teil
- 36: Verschlusselement
- 38: Zuführ- und Entnahmeöffnung
- 40, 42, 44: Sensorelement
- 46, 47: Bildsensor
- 48: Steuereinheit
- 50, 52: Beobachtungsoptik
- 51, 53, 54, 55, 57,: Distales Ende
- 59, 61 62: Strahlteiler
- 70: Ausgabeeinheit
- 74: Bereich
- 80, 82: Optische Elemente
- P1: Richtung

## Patentansprüche

1. Anordnung zur sterilen Handhabung eines nicht sterilen Endoskops in einer sterilen Umgebung,
mit einer sterilen Endoskophülle (14), die ein an einem distalen Ende der Endoskophülle (14) angeordnetes optisches Element (32) hat, wobei das optische Element (32) elektromagnetische Strahlung in einem Absorptionswellenlängenbereich absorbiert, der im mittleren infraroten Wellenlängenbereich von 3 µm bis 50 µm liegt, und
mit einem nicht sterilen Endoskop (12), das einen Endoskopschaft (20) und ein an einem distalen Ende des Endoskopschafts (20) angeordnetes optischen Element umfasst,
wobei das optische Element des Endoskops für elektromagnetische Strahlung in dem Absorptionswellenlängenbereich transparent ist,
wobei das Endoskop (12) in der Endoskophülle (14) aufgenommen ist und durch diese gegenüber der Umgebung steril abgeschirmt ist,
wobei das Endoskop (12) ein erstes Sensorelement (40) hat, das eine von dem optischen Element (32) der Endoskophülle (14) ausgehende elektromagnetische Strahlung in dem Absorptionswellenlängenbereich erfasst,
wobei der Endoskopschaft (20) einen ersten Lichtwellenleiter (56) enthält, der mit dem ersten Sensorelement (40) optisch verbunden ist und der in das distale Ende des Endoskopschafts (20) einfallende elektromagnetische Strahlung in dem Absorptionswellenlängenbereich zu dem ersten Sensorelement (40) leitet,
**dadurch gekennzeichnet, dass**
das Endoskop (40) ein zweites Sensorelement (42) hat, das elektromagnetische Strahlung in dem Absorptionswellenlängenbereich erfasst,
wobei der Endoskopschaft (20) einen zweiten Lichtwellenleiter (58) enthält, der mit dem zweiten Sensorelement (42) optisch verbunden ist und der elektromagnetische Strahlung in dem Absorptionswellenlängenbereich von dem distalen Ende des Endoskopschafts (20) zu dem zweiten Sensorelement (42) leitet, und
wobei der zweite Lichtwellenleiter (58) an einem distalen Ende (59) optisch verschlossen ist.

2. Anordnung nach Anspruch 1, **dadurch gekennzeichnet, dass** das optische Element (32) in wenigstens einem optischen Wellenlängenbereich außerhalb des Absorptionswellenlängenbereichs transparent ist.

3. Anordnung nach Anspruch 1 oder 2, **dadurch gekennzeichnet, dass** der Absorptionswellenlängenbereich ein Wellenlängenbereich von 9 µm bis 10 µm, von 8 µm bis 12 µm oder von 8 µm bis 14 µm ist.

4. Anordnung nach einem der vorhergehenden Ansprüche, **gekennzeichnet durch** eine Steuereinheit (48), die auf Grundlage der durch das erste Sensorelement (40) erfassten elektromagnetischen Strahlung eine Temperatur des optischen Elements (32) der Endoskophülle ermittelt.

5. Anordnung nach Anspruch 4, **gekennzeichnet durch** eine Ausgabeeinheit (70), die ein akustisches oder optisches Warnsignal ausgibt, wenn die mithilfe der Steuereinheit (48) ermittelte Temperatur des optischen Elements (32) der Endoskophülle (14) einen voreingestellten Wert erreicht und/oder überschreitet.

6. Anordnung nach einem der vorhergehenden Ansprüche , **dadurch gekennzeichnet, dass** das Endoskop (12) einen Strahlteiler (62) aufweist, der die elektromagnetische Strahlung in dem Absorptionswellenlängenbereich aus einer Beobachtungsoptik (50) des Endoskops (12) auskoppelt und auf das erste Sensorelement (40) lenkt.

7. Anordnung nach einem der vorhergehenden Ansprüche , **gekennzeichnet durch** eine oder die Steuereinheit (48), die auf Grundlage der durch das erste Sensorelement (40) erfassten elektromagnetischen Strahlung eine Temperatur des optischen Elements (32) der Endoskophülle (14) ermittelt und die auf Grundlage der durch das zweite Sensorelement (42) erfassten elektromagnetischen Strahlung eine Temperatur des optischen Elements des Endoskops (12) ermittelt.

8. Anordnung nach Anspruch 7, **gekennzeichnet durch** eine oder die Ausgabeeinheit (70), die ein akustisches und/oder optisches Warnsignal ausgibt, wenn die ermittelte Temperatur des optischen Elements (32) der Endoskophülle (14) und die ermittelte Temperatur des optischen Elements des Endoskops (12) einen jeweils voreingestellten Wert erreichen und/oder überschreiten

9. Anordnung nach einem der vorhergehenden Ansprüche, **dadurch gekennzeichnet, dass** das optische Element (32) der Endoskophülle (14) wenigstens einen Bereich (74) aufweist, der für elektromagnetische Strahlung in dem Absorptionswellenlängenbereich transparent ist.

10. Anordnung nach Anspruch 9, **dadurch gekennzeichnet, dass** das Endoskop (12) ein drittes Sensorelement (44) hat und dass der Endoskopschaft (20) einen dritten Lichtwellenleiter (60) enthält, der mit dem dritten Sensorelement (44) optisch verbunden ist, wobei ein distales Ende (51) des dritten Lichtwellenleiters (60) dem wenigstens einen Bereich (74) gegenüberliegend angeordnet ist, und der in das distale Ende (61) des dritten Lichtwellenleiters (60) einfallende elektromagnetische Strahlung in dem Absorptionswellenlängenbereich zu dem dritten Sensorelement (44) leitet.

## Claims

1. An arrangement for the sterile handling of a non-sterile endoscope in a sterile environment,
with a sterile endoscope sheath (14) having an optical element (32) arranged at a distal end of the endoscope sheath (14),
wherein the optical element (32) absorbs electromagnetic radiation in an absorption wavelength range lying in the mid-infrared wavelength range from 3 µm to 50µm, and
with a non-sterile endoscope (12) comprising an endoscope shaft (20) and an optical element arranged at a distal end of the endoscope shaft (20),
wherein the optical element of the endoscope is transparent to electromagnetic radiation in the absorption wavelength range, and wherein the endoscope (12) is received in the endoscope sheath (14) and is shielded by it against the environment in a sterile manner,
wherein the endoscope (12) has a first sensor element (40) which detects an electromagnetic radiation in the absorption wavelength range originating from the optical element (32) of the endoscope sheath (14),
wherein the endoscope shaft (20) includes a first optical fiber (56) which is optically connected to the first sensor element (40) and which guides electromagnetic radiation in the absorption wavelength range entering the distal end of the endoscope shaft (20) to the first sensor element (40),
**characterized in that**
the endoscope (40) has a second sensor element (42) that detects electromagnetic radiation in the absorption wavelength range,
wherein the endoscope shaft (20) includes a second optical fiber (58) which is optically connected to the second sensor element (42) and which guides electromagnetic radiation in the absorption wavelength range from the distal end of the endoscope shaft (20) to the second sensor element (42), and
wherein the second optical fiber (58) is optically closed at a distal end (59).

2. The arrangement according to claim 1, **characterized in that** the optical element (32) is transparent in at least one optical wavelength range outside the absorption wavelength range.

3. The arrangement according to claim 1 or 2, **characterized in that** the absorption wavelength range is a wavelength range from 9 µm to 10 µm, from 8 µm to 12 µm or from 8 µm to 14 µm.

4. The arrangement according to any one of the preceding claims, **characterized by** a control unit (48) which determines a temperature of the optical element (32) of the endoscope sheath on the basis of the electromagnetic radiation detected by the first sensor element (40).

5. The arrangement according to claim 4, **characterized by** an output unit (70) which outputs an acoustic or optical warning signal when the temperature of the optical element (32) of the endoscope sheath (14) determined with the aid of the control unit (48) reaches and/or exceeds a preset value.

6. The arrangement according to any one of the preceding claims, **characterized in that** the endoscope (12) has a beam splitter (62) which couples the electromagnetic radiation in the absorption wavelength range out of an observation optical system (50) of the endoscope (12) and directs it onto the first sensor element (40).

7. The arrangement according to any one of the preceding claims, **characterized by** a or the control unit (48) which determines a temperature of the optical element (32) of the endoscope sheath (14) on the basis of the electromagnetic radiation detected by the first sensor element (40) and which determines a temperature of the optical element of the endoscope (12) on the basis of the electromagnetic radiation detected by the second sensor element (42).

8. The arrangement according to claim 7, **characterized by** a or the output unit (70) which outputs an acoustic and/or optical warning signal when the determined temperature of the optical element (32) of the endoscope sheath (14) and the determined temperature of the optical element of the endoscope (12) each reach and/or exceed a preset value.

9. The arrangement according to any one of the preceding claims, **characterized in that** the optical element (32) of the endoscope sheath (14) has at least one area (74) which is transparent to the electromagnetic radiation in the absorption wavelength range.

10. The arrangement according to claim 9, **characterized in that** the endoscope (12) has a third sensor element (44) and that the endoscope shaft (20) includes a third optical fiber (60) which is optically connected to the third sensor element (44), wherein a distal end (51) of the third optical fiber (60) is arranged opposite to the at least one area (74), and which guides electromagnetic radiation in the absorption wavelength range entering the distal end (61) of the third optical fiber (60) to the third sensor element (44).

## Revendications

1. Dispositif permettant une manipulation stérile d'un endoscope non stérile dans un environnement stérile,
avec une gaine d'endoscope stérile (14) présentant un élément optique (30) agencé à une extrémité distale de la gaine d'endoscope (14),
dans lequel l'élément optique (32) absorbe un rayonnement électromagnétique dans une plage de longueurs d'onde d'absorption située dans la plage de longueurs d'onde de l'infrarouge moyen comprise entre 3 µm et 50 µm, et
avec un endoscope non stérile (12) comprenant une tige d'endoscope (20) et un élément optique agencé à une extrémité distale de la tige d'endoscope (20),
dans lequel l'élément optique de l'endoscope est transparent à un rayonnement électromagnétique dans la plage de longueurs d'onde d'absorption,
dans lequel l'endoscope (12) est accueilli dans la gaine d'endoscope (14) et est protégé de manière stérile par rapport à l'environnement grâce à ladite gaine d'endoscope,
dans lequel l'endoscope (12) présente un premier élément formant capteur (40) qui détecte un rayonnement électromagnétique émanant de l'élément optique (32) de la gaine d'endoscope (14) dans la plage de longueurs d'onde d'absorption,
dans lequel la tige d'endoscope (20) contient un premier guide d'onde optique (56) qui est optiquement relié au premier élément formant capteur (40) et qui guide vers le premier élément formant capteur (40) le rayonnement électromagnétique dans la plage de longueurs d'onde d'absorption pénétrant par l'extrémité distale de la tige d'endoscope (20),
**caractérisé en ce que**
l'endoscope (40) présente un deuxième élément formant capteur (42) qui détecte un rayonnement électromagnétique dans la plage de longueurs d'onde d'absorption,
dans lequel la tige d'endoscope (20) contient un deuxième guide d'onde optique (58) qui est optiquement relié au deuxième élément formant capteur (42) et qui guide le rayonnement électromagnétique dans la plage de longueurs d'onde d'absorption de l'extrémité distale de la tige d'endoscope (20) vers le deuxième élément formant capteur (42), et
dans lequel le deuxième guide d'onde optique (58) est optiquement fermé au niveau d'une extrémité distale (59).

2. Dispositif selon la revendication 1, **caractérisé en ce que** l'élément optique (32) est transparent dans au moins une plage de longueurs d'onde optique située en dehors de la plage de longueurs d'onde d'absorption.

3. Dispositif selon la revendication 1 ou 2, **caractérisé en ce que** la plage de longueurs d'onde d'absorption est une plage de longueurs d'onde comprise entre 9 µm et 10 µm, entre 8 µm et 12 µm ou entre 8 µm et 14 µm.

4. Dispositif selon l'une quelconque des revendications précédentes, **caractérisé par** une unité de commande (48) qui détermine une température de l'élément optique (32) de la gaine d'endoscope en se basant sur le rayonnement électromagnétique détecté grâce au premier élément formant capteur (40).

5. Dispositif selon la revendication 4, **caractérisé par** une unité de sortie (70) qui émet un signal d'avertissement acoustique ou optique lorsque la température, déterminée par l'unité de commande (48), de l'élément optique (32) de la gaine d'endoscope (14) atteint et/ou dépasse une valeur prédéfinie.

6. Dispositif selon l'une quelconque des revendications précédentes, **caractérisé en ce que** l'endoscope (12) présente un diviseur de faisceau (62) qui extrait d'une optique d'observation (50) de l'endoscope (12) le rayonnement électromagnétique dans la plage de longueurs d'onde d'absorption et le renvoie sur le premier élément formant capteur (40).

7. Dispositif selon l'une quelconque des revendications précédentes, **caractérisé par** une unité de commande ou l'unité de commande (48) qui détermine une température de l'élément optique (32) de la gaine d'endoscope (14) en se basant sur le rayonnement électromagnétique détecté grâce au premier élément formant capteur (40) et qui détermine une température de l'élément optique de l'endoscope (12) en se basant sur le rayonnement électromagnétique détecté grâce au deuxième élément formant capteur (42).

8. Dispositif selon la revendication 7, **caractérisé par** une unité de sortie ou l'unité de sortie (70) qui émet un signal d'avertissement acoustique et/ou optique lorsque la température déterminée de l'élément optique (32) de la gaine d'endoscope (14) et la température déterminée de l'élément optique de l'endoscope (12) atteignent et/ou dépassent une valeur respectivement prédéfinie.

9. Dispositif selon l'une quelconque des revendications précédentes, **caractérisé en ce que** l'élément optique (32) de la gaine d'endoscope (14) présente au moins une région (74) qui est transparente au rayonnement électromagnétique dans la plage de longueurs d'onde d'absorption.

10. Dispositif selon la revendication 9, **caractérisé en ce que** l'endoscope (12) présente un troisième élément formant capteur (44) et **en ce que** la tige d'endoscope (20) contient un troisième guide d'onde optique (60) qui est optiquement relié au troisième élément formant capteur (44), dans lequel une extrémité distale (51) du troisième guide d'onde optique (60) est agencée face à la au moins une région (74), et qui guide vers le troisième élément formant capteur (44) le rayonnement électromagnétique dans la plage de longueur d'onde d'absorption pénétrant par l'extrémité distale (61) du troisième guide d'onde optique (60).
